Europäisches Patentamt

(19) European Patent Office

Office européen des brevets

(11) EP 0 889 054 B1

(12) **EUROPÄISCHE PATENTSCHRIFT**

(45) Veröffentlichungstag und Bekanntmachung des Hinweises auf die Patenterteilung:
**08.10.2003  Patentblatt 2003/41**

(51) Int Cl.⁷: **C07K 16/28**, A61K 39/395, C12P 21/08, C12N 5/18, C07K 14/705, G01N 33/574

(21) Anmeldenummer: **98108845.3**

(22) Anmeldetag: **15.05.1998**

(54) **Antikörper 67D2 gegen CD164**

Antibody 67D2 against CD164

Anticorps 67D2 contre CD164

(84) Benannte Vertragsstaaten:
**AT BE CH DE FR GB LI**

(30) Priorität: **30.06.1997  DE 19727813**

(43) Veröffentlichungstag der Anmeldung:
**07.01.1999  Patentblatt 1999/01**

(73) Patentinhaber: **Eberhard-Karls-Universität Tübingen Universitätsklinikum**
**72076 Tübingen (DE)**

(72) Erfinder:
• **Bühring, Hans-Jörg, Dr.**
  **72076 Tübingen (DE)**
• **Watt, Suzanne M., Dr.**
  **Ealing, London W13 8EN (GB)**

(74) Vertreter: **Otten, Hajo, Dr.-Ing. et al**
**Witte, Weller & Partner**
**Patentanwälte,**
**Rotebühlstrasse 121**
**70178 Stuttgart (DE)**

(56) Entgegenhaltungen:
**DE-C- 19 530 272        DE-C- 19 530 273**

• ZANNETTINO A C W ET AL: "IDENTIFICATION & FUNCTIONAL CLONING OF MGC-24, A MUCIN-LIKE MOLECULE EXPRESSED BY HAEMOPOIETIC PROGENITORS AND BONE MARROW STROMAL CELLS: A NEGATIVE REGULATOR OF HAEMOPOIESIS" BLOOD, Bd. 86, Nr. 10, SUPPL. 01, 15. November 1995 (1995-11-15), Seite 591A XP002018705 PHILADELPHIA US ISSN: 0006-4971

• BÜHRING H.-J. ET AL: "PEANUT AGGLUTININ BINDING PROTEIN MGC-24 IS A MUCIN PREFERENTIALLY EXPRESSED ON ERYTIIROID CELLS AND A SUBSET OF CD34+ BONE MARROW CELLS " BLOOD , Bd. 86, Nr. 10, SUPPL. 01, 15. November 1995 (1995-11-15), Seite 659a XP000606397 PHILADELPHIA, US ISSN: 0006-4971

• PANCINO G ET AL: "A novel monoclonal antibody (7B10) with differential reactivity between human and mammary carcinoma and normal breast" CANCER RESEARCH., Bd. 47, 15. August 1987 (1987-08-15), Seiten 4444-4452, XP000989760 BALTIMORE, US ISSN: 0008-5472

• PANCINO G.F. ET AL.: "Characterization and distribution in normal and tumoral rissues of breast cancer-associated antigen defined by monoclonal antibody 7B10" CANCER RESEARCH., Bd. 49, 15. Dezember 1989 (1989-12-15), Seiten 7078-7085, XP000989757 BALTIMORE, US ISSN: 0008-5472

• HILKENS J ET AL.: "Monoclonal antibodies against human milk-fat globule membranes detecting differentiation antigens on the mammary gland and its tumours" INTERNATIONAL JOURNAL OF CANCER, Bd. 34, 1984, Seiten 197-206, XP000926227

• ZOTTER S ET AL: "EPITHELIAL MARKERS FOR PARAFFIN-EMBEDDED HUMAN TISSUES IMMUNOHISTOCHEMISTRY WITH MONOCLONAL ANTIBODIES AGAINST MILK FAT GLOBULE ANTIGENS" VIRCHOWS ARCHIV A PATHOLOGICAL ANATOMY AND HISTOPATHOLOGY, Bd. 406, Nr. 2, 1985, Seiten 237-251, XP000926230 ISSN: 0174-7398

• DOYONNAS R ET AL.: "CD164 monoclonal antibodies that block hemopoietic progenitor cell adhesion and proliferation interact with the first mucin domain of the CD164 receptor" JOURNAL OF IMMUNOLOGY., Bd. 165, Nr. 2, 15. Juli 2000 (2000-07-15), Seiten 840-851, XP000990011 BALTIMORE US ISSN: 0022-1767

## Beschreibung

Die Erfindung betrifft einen spezifisch gegen CD164 gerichteten monoklonalen Antikörper.

Derartige Antikörper sind aus der DE 195 30 272 C1 und der DE 195 30 273 C1 bekannt.

CD164 ist ein glykosyliertes Zelloberflächenprotein, das ursprünglich als Peanut Agglutinin (PNA) -bindendes Glykoprotein identifiziert wurde.

Maligne Zellen von vielen menschlichen Tumoren besitzen an ihrer Zelloberfläche Peanut Agglutinin (PNA)-bindende Glykoproteine. Diese PNA-bindenden Glykoproteine bieten u.a. die Möglichkeit, Nachweisreagenzien und/oder therapeutisch wirksame Reagenzien direkt an die betreffenden Zellen heranzuführen und daran zu binden. Seit einigen Jahren bekannt sind die nach der inzwischen eingeführten MUC-Nomenklatur benannten Glykoproteine MUC1, MUC2 und MUC3. Ein kürzlich identifiziertes Zelloberflächen-Glykoprotein ist das Protein MGC-24 (Multi-Glycosylated Core protein of 24kDa), dessen Aminosäuresequenz und dazugehörige Nukleotidsequenz von Masuzawa, et al., J. BIOCHEM. 112, 609 - 615, (1992) vollständig aufgeklärt wurde.

In jüngster Zeit wurde eine Variante oder Isoform des Proteins MGC-24 gefunden, die MGC-24v genannt wurde. MGC-24 und MGC-24v weisen fast identische extrazelluläre Domänen auf. MGC-24 ist jedoch ein vornehmlich sezerniertes Protein, während MGC-24v ein integrales Membranprotein vom Typ I ist. Mit seinem Molekulargewicht von 160 kD ist MGC-24v ein Homodimer aus zwei 80 kD-Untereinheiten.

In einer CD-Klassifizierung wurde dem Glykoprotein MGC-24v die Bezeichnung CD164 zugewiesen, die im folgenden verwendet werden soll.

Die Funktion von CD164 ist noch völlig ungeklärt. Dieses Zelloberflächenprotein wird jedoch auf einer Reihe neoplastischer Gewebe epithelialen Ursprungs exprimiert. Daher eignet es sich besonders gut als Angriffspunkt für eine gezielte zelluläre Diagnose und Therapie derartiger Tumoren.

Als Vermittler für ein entsprechendes zellulär zielgerichtetes Diagnostikum bzw. Therapeutikum kommt insbesondere ein Antikörper in Betracht, der spezifisch an CD164 bindet.

Ein solcher Antikörper kann sowohl mit einfachen Nachweisreagenzien wie Fluoreszenzfarbstoffen oder radioaktiven Stoffen als auch mit speziellen therapeutisch wirksamen Reagenzien gekoppelt sein.

Aus der DE 195 30 272 C1 ist ein monoklonaler Antikörper bekannt, der an MGC-24 bindet und die Bezeichnung 103B2 trägt. Ein weiterer, gegen MGC-24 gerichteter Antikörper ist in der DE 195 30 273 C1 offenbart. Dieser Antikörper trägt die Bezeichnung 105A5. In der Zwischenzeit stellte sich heraus, daß beide Antikörper auch die Isoform MGC-24v, also CD164, erkennen (Zannettino et al., "CD164 Workshop Panel Report", in: Leucocyte Typing VI, Kishimoto, T. et al., Garland Publishing, New York (im Druck)).

Generell wurden bereits früher monoklonale Antikörper entwickelt, die an tumorassoziierte Glykoproteine binden. Bspw. bindet ein mit $7B_{10}$ bezeichneter Antikörper an ein nicht näher charakterisiertes Glykoprotein von 76 kDa, das in verschiedenen Tumorgeweben vorhanden zu sein scheint (vgl. Pancino et al., Cancer Research 47, 4444-4452 (1987); Pancino et al., Cancer Research 49, 7078-7085 (1989)).

Zum Nachweis und zur gezielten Beeinflussung des Antigens CD164 ist es wünschenswert, daß verschiedene monoklonale Antikörper zur Verfügung stehen, da so Ergebnisse mit hoher Zuverlässigkeit experimentell bestätigt werden können.

Darüber hinaus können sich verschiedene, gegen das gleiche Antigen gerichtete monoklonale Antikörper in ihren jeweiligen Sensitivitäten und Kreuzreaktivitäten unterscheiden. Wenn mehrere monoklonale Antikörper gegen ein gegebenes Antigen zur Verfügung stehen, so kann in Abhängigkeit von der Verwendung der am besten geeignete Antikörper ausgewählt werden.

Der in der DE 195 30 272 C1 beschriebene Antikörper 103B2 gehört der Immunglobulinklasse IgG3 an, wohingegen der in der DE 195 30 273 C1 offenbarte Antikörper 105A5 zur Immunglobulinklasse IgGM gehört.

Antikörper, also Immunglobuline, bestehen aus variablen Regionen, deren Funktion die spezifische Epitoperkennung ist, sowie aus konstanten Regionen, die charakteristisch für eine Immunglobulinklasse sind. Auch die konstanten Regionen der Antikörper beeinflussen ihre Eigenschaften wesentlich. So bilden Antikörper, die zur Immunglobulinklasse IgM gehören, tetramere Strukturen aus, während die zur IgG-Klasse gehörenden Antikörper monomer vorliegen. Außerdem gibt es vier verschiedene Subklassen der Immunglobulinklasse IgG, die sich hinsichtlich ihrer Disulfidbrückenbindung, ihrer Stabilität und weiterer physikochemischer Eigenschaften unterscheiden.

Die Verschiedenartigkeit der konstanten Regionen der Immunglobuline nutzt man vor allem bei den heute allgemein üblichen indirekten Nachweismethoden, bei denen Antikörper durch sekundäre, an Nachweisreagenzien gekoppelte Antikörper nachgewiesen werden. Diese sekundären Antikörper binden spezifisch an die konstanten Regionen der primären, ihrerseits an die Antigene bindenden Antikörper. Sekundäre Antikörper erkennen somit meist nur eine Immunglobulinklasse.

Es ist daher zweckmäßig, zu verschiedenen Immunglobulinklassen gehörende monoklonale Antikörper zur Verfügung zu haben, um ein möglichst breites Spektrum an Nachweismöglichkeiten anwenden zu können.

Der vorliegenden Erfindung liegt daher die Aufgabe zugrunde, einen weiteren monoklonalen Antikörper bereitzustellen, der spezifisch an CD164 bindet und der in praktisch unbegrenzter Menge zur Verfügung steht.

Diese Aufgabe wird durch die Bereitstellung eines monoklonalen Antikörpers gelöst, der von Hybridomzellen produziert und freigesetzt wird, die unter der Nummer DSM ACC2303 bei der Deutschen Sammlung von Mikroorganismen und Zellkulturen GmbH, DMSZ, Mascheroder Weg 1b, 38124 Braunschweig, hinterlegt sind. Er ist mit der Bezeichnung 67D2 benannt.

Im Stand der Technik ist ein ebenfalls mit "67D2" bezeichneter monoklonaler Antikörper bekannt (Hilkens et al., Int.J.Cancer: 34, 197-206 (1984); Zotter et al., Virchows Arch [Pathol. Anat.]: 406, 237-251 (1985)), der jedoch ein gänzlich unterschiedliches Antigen erkennt, einen MAM-5 genannten Oberflächenmarker auf Membranen des humanen Milchfettglobulins, so dass mit dem erfindungsgemäßen Antikörper keinerlei Zusammenhang besteht.

Im Laufe der Analysen zu dem erfindungsgemäßen monoklonalen Antikörper 67D2 wurde nachgewiesen, daß er zur Immunglobulinklasse IgG1 gehört (siehe dazu auch Beispiel 1). Somit wird mit diesem monoklonalen Antikörper ein Antikörper mit einem bisher nicht verfügbaren Subtyp bereitgestellt, der standardisiert reproduzierbar ist, potentiell unbegrenzt hergestellt werden kann und der spezifisch an das Zelloberflächenglykoprotein CD164 bindet.

Der erfindungsgemäße Antikörper ermöglicht eine gezielte Erkennung und Beeinflussung von Zellen, die CD164 exprimieren. Er stellt damit ein weiteres, vielseitig einsetzbares Mittel für den Arzt und Forscher dar, um einerseits solche Zellen nachzuweisen, und zwar sowohl in der Zellkultur als auch im Patientenorganismus, und um andererseits diese Zellen ggf. zu manipulieren, entweder durch den Antikörper selbst oder durch daran oder an geeignete sekundäre Antikörper gekoppelte spezifische Reagenzien.

Die Erfindung betrifft ferner Hybridomzellen, die unter der Nr. DSM ACC2303 bei der Deutschen Sammlung von Mikroorganismen und Zellkulturen GmbH, DMSZ, hinterlegt sind und den Antikörper mit der Bezeichnung 67D2 produzieren.

Darüber hinaus wird ein Verfahren zur Herstellung von Hybridomzellen beschrieben, die einen Antikörper gegen das Zelloberflächenglykoprotein CD164 synthetisieren und freisetzen. Dieses Verfahren umfaßt die im Stand der Technik grundsätzlich geläufigen Schritte, wie sie bspw. von Bühring et al. in Hybridoma 1991, Band 10, Nr. 1, S. 77-78 beschrieben wurden:

1. Immunisierung oder Sensibilisierung eines Tieres, vorzugsweise einer Maus vom Balb/c-Stamm, mit dem Antigen bzw. Immunogen;

2. Gewinnung der antikörperproduzierenden Zellen, vorzugsweise der Milzlymphozyten dieses Tieres;

3. Fusionierung dieser antikörperproduzierenden Zellen mit einer stabilen, immortalisierten Zellinie, vorzugsweise einer Myelomzellinie, zu Hybridomzellen; und

4. Vereinzelung und Vermehrung (Klonierung) solcher Hybridomzellen, die einen Antikörper sezernieren, der an das Antigen bindet.

Das Verfahren zeichnet sich dadurch aus, daß das Tier mit Zellen der Brustkarzinomzellinie T-47D (American Type Culture Collection, ATCC Nr. HTB133) immunisiert wird.

Dabei erwies sich als Vorteil, daß diese Zellinie eine starke Expression von CD164 zeigt, wie sich während der zu dem Antikörper 67D2 führenden Versuche zeigte.

Wenn Hybridomzellen gesucht werden, die gegen CD164 gerichtete Antikörper produzieren, so ist es bei der Vereinzelung der Hybridomzellen bevorzugt, wenn solche Hybridomzellen ausgewählt werden, die Antikörper mit einer Spezifität für eine Zellinie produzieren, die zuvor mit der cDNA für CD164 transfiziert wurde.

Die Herstellung einer solchen Zellinie ist in Ausführungsbeispiel 2 beschrieben.

Diese transfizierte Zellinie ist zur Selektion der Hybridomzellen besonders vorteilhaft, da sie CD164 in großen Mengen auf ihrer Zelloberfläche exprimiert.

Die Erfindung betrifft auch die Verwendung des erfindungsgemäßen Antikörpers zur diagnostischen und/oder therapeutischen Behandlung von Tumoren, insbesondere von Magen-, Colonund Brustkarzinomen.

Tumorzellen, insbesondere Zellen von Magen-, Colon- und Brustkarzinomen, zeichnen sich durch einen vergleichsweise hohen Gehalt an Zelloberflächenglykoprotein CD164 aus. Ein erfindungsgemäßer Antikörper, der mit einem Nachweismittel, beispielsweise einem radioaktiven Marker, gekoppelt ist, bindet dieses Nachweismittel indirekt an diese Zellen und ermöglicht damit den direkten Nachweis dieser Zellen, beispielsweise mit röntgendiagnostischen /szintigraphischen Methoden. Damit ist eine sehr frühe Tumordiagnose unter Umständen sogar *in vivo* möglich.

In entsprechender Art und Weise kann der Antikörper mit einem therapeutisch wirksamen Mittel gekoppelt sein und dadurch eine direkte und gezielte Beeinflussung oder gar Eliminierung von CD164 tragenden Zellen, insbesondere Tumorzellen, ermöglichen.

In einer bevorzugten Ausführungsform wird ein Antikörper, der von den unter der Nummer DSM ACC2303 bei der Deutschen Sammlung von Mikroorganismen und Zellkulturen GmbH, DSMZ, hinterlegten Hybridomzellen produziert und freigesetzt wird, zu einer solchen diagnostischen und/oder therapeutischen Behandlung verwendet.

Um die therapeutische und/oder diagnostische Applikation des erfindungsgemäßen Antikörpers zu erleichtern, kann der Antikörper mit entsprechend geeigneten Hilfssubstanzen in einer pharmazeutischen Zubereitung vermischt sein. Die Erfindung betrifft deshalb auch ein pharmazeutisches Mittel zur diagnostischen und/oder therapeutischen Behandlung von Tumoren, das den erfindungsgemäßen, an CD164 bindenden Antikörper enthält, der von den unter der Nummer DSM ACC2303 bei der Deutschen Sammlung von Mikroorganismen und Zellkulturen GmbH, DSMZ, hinterlegten Hybridomzellen produziert und freigesetzt wird.

Mit dem erfindungsgemäßen Antikörper können CD164 tragende Zellen aus einer Suspension verschiedenartiger Zellen mit den im Stand der Technik bekannten Testverfahren, wie z.B. dem Enzyme linked immunosorbent assay, kurz ELISA, oder dem Radioimmunoassay, kurz RIA, nachgewiesen werden. Die vorliegende Erfindung betrifft deshalb auch einen Kit zum Nachweis des Zelloberflächenglykoproteins CD164, der den Antikörper 67D2 umfaßt.

Im Rahmen der vorliegenden Erfindung wurde überraschenderweise gefunden, daß der monoklonale Antikörper mit der Bezeichnung 67D2, der von den unter der Nummer DSM ACC2303 bei der Deutschen Sammlung von Mikroorganismen und Zellkulturen GmbH, DSMZ, hinterlegten Hybridomzellen erzeugt wird, an eine Subpopulation mononukleärer Knochenmarkszellen bindet.

Die Erfindung betrifft deshalb auch die Verwendung des Antikörpers 67D2 zum Nachweis von hämatopoetischen Zellen, sowie einen Kit zum Nachweis von hämatopoetischen Zellen, der den Antikörper 67D2 enthält. Dadurch ist es möglich, undifferenzierte $CD34^+CD34^+$-Subpopulationen für funktionelle Analysen aus dem Knochenmark zu selektieren und aufzureinigen.

Weitere Vorteile ergeben sich aus der nachstehenden Beschreibung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird im folgenden anhand von Anwendungs- und Ausführungsbeispielen näher erläutert.

Beispiel 1: Herstellung und Charakterisierung von monoklonalen Antikörpern gegen das Zelloberflächenglykoprotein CD164

Als Antigen werden Zellen der Brustkarzinomzellinie T-47D verwendet, die unter der ATCC Nr. HTB133 kommerziell erhältlich sind.

Acht Wochen alte Balb/c-Mäuse werden zweimal in Intervallen von 10 Tagen intraperitoneal mit $10^7$ Zellen der Zellinie T-47D immunisiert. Vier Tage vor der Fusion werden 5 x $10^5$ Zellen direkt in die Milz appliziert, um die Immunantwort zu verstärken.

Die Antikörper-Bildung im Mausorganismus wird dadurch überprüft, daß das Blutserum des betreffenden Tieres in dem dem Fachmann geläufigen ELISA-Test auf Bindungseigenschaften mit dem Antigen untersucht wird.

Nach ca. 3 Wochen werden die Lymphozyten des erfolgreich immunisierten Tieres gewonnen, indem die Milz herausoperiert und zu einer Zellsuspension zerkleinert wird.

Die suspendierten Milzzellen werden in Anwesenheit von Polyethylenglykol mit Myelomzellen des bekannten Stammes SP2/0 fusioniert. Die Fusionskultur wird in Hypoxanthin-, Aminopterinund Thymidin-(HAT-)haltigem Medium, hier in HAT-RPMI-1640, kultiviert, in dem sich nur Hybridzellen vermehren können, da diese sowohl die Eigenschaft der Myelomzellen zur unbegrenzten Teilungsfähigkeit als auch die Eigenschaft der Antikörper produzierenden Lymphozyten zum Wachstum in HAT-haltigem Medium haben.

Nach der Fusion werden die Zellen in Napfplatten ausplattiert und bei 37 °C, 5 % $CO_2$ inkubiert.

Die Kulturüberstände werden nach 10-14 Tagen auf der Zellinie T-47D im Durchflußzytometer gescreent. In einem zweiten Schritt werden die Überstände auf Reaktivität mit einer Zellinie getestet, die zuvor mit der cDNA für CD164 transfiziert worden war. Die Herstellung dieser transfizierten Zellinie ist in Beispiel 2 beschrieben. Hybridome, die Antikörper mit Spezifität für diese Zellinie produzieren, werden ausgewählt und nach dem bekannten Grenzverdünnungsverfahren vereinzelt und kultiviert, d.h. kloniert.

Bei dieser Screening-Strategie wird Nutzen aus der Tatsache gezogen, daß die transfizierte Zellinie CD164 in großen Mengen auf ihrer Zelloberfläche exprimiert.

Positiv reagierende Hybridomzellkulturen werden weiter kultiviert, die Antikörper angereichert, gereinigt und charakterisiert. Nach der obigen Screening-Strategie wurde der monoklonale Antikörper 67D2 erhalten. Der Isotyp wurde über PE-konjugierte Anti-Isotyp-spezifische Antiseren durch direkte Immunfluoreszenz zu IgG1 bestimmt.

Die Produktion, Reinigung und Charakterisierung der Antikörper erfolgte mit den in Fachkeisen allgemein bekannten Methoden.

Der Antikörper 67D2, der von den unter der Nummer DSM ACC2303 bei der Deutschen Sammlung von Mikroorganismen und Zellkulturen GmbH, DSMZ, hinterlegten Hybridomzellen erzeugt wird, weist die folgenden cha-

rakteristischen Merkmale auf:

| Immunglobulinklasse | IgGI |
|---|---|
| spezifische Bindung an | CD164 |

Beispiel 2: Herstellung einer CD164 exprimierenden Zellinie

Während der Untersuchungen, die zur Identifizierung des Antigens des monoklonalen Antikörpers 103B2 (DE 195 30 272 C1) führten, wurde eine CD164 exprimierende Zellinie etabliert. Diese Untersuchungen zielten darauf ab, das entsprechende Gen zu finden und zu analysieren.

Um das Gen zu isolieren, das für das Antigen codiert, das von dem monoklonalen Antikörper 103B2 erkannt wird, wurde eine retrovirale Expressionsbibliothek nach dem Verfahren erstellt, das von Rayner und Gonda in Mol. Cell.Biol. 1994, Band 14, Seite 880 beschrieben wurde. Bei dem hier verwendeten Verfahren wurde mRNA aus kultivierten Stromazellen des menschlichen Knochenmarks verwendet.

cDNA-Transkripte wurden direkt in den retroviralen Plasmid-Vektor pRUF.Neo kloniert. DNA aus der Bibliothek wurde verwendet, um eine amphotrope Wirtszellinie (PA317) zu transfizieren. übergangsweise erzeugte retrovirale Partikel wurden geerntet und dazu verwendet, eine ecotrope Wirtszellinie stabil zu infizieren. Von diesen Zellen produzierte Viren wurden daraufhin verwendet, um die faktorabhängige hämatopoetische murine Zellinie FDC-P1 zu infizieren.

Infizierte FDC-P1-Zellen wurden bezüglich der G418-Resistenz ausgewählt, woraufhin Zellen isoliert und angereichert wurden, die das von dem Antikörper 103B2 erkannte Antigen exprimieren. Die Anreicherung von Zellen, die von dem Antikörper erkannt wurden, erfolgte dabei durch Einsatz mehrerer Durchläufe einer immuno-magnetischen Zellsortierung (Dynabeads). Nach dieser FACS-Sortierung wurden klonale Populationen der transfizierten Zellen etabliert.

Daraufhin wurden die proviralen cDNA-Inserts von genomischer DNA der infizierten Zellen zurückgewonnen. Zu diesem Zweck wurde eine PCR-Amplifikation durchgeführt, wobei spezifische retrovirale Primer verwendet wurden, die die Klonierungsstelle in dem Plasmidvektor flankieren. Auf diese Weise war es möglich, ein cDNA-Insert von ungefähr 3 kBp zu isolieren.

Eine Sequenzanalyse ergab, daß dieses Insert eine Isoform eines zuvor klonierten Gens identifizierte. Das zuvor klonierte Gen gehört zu der mucin-Familie und codiert für MGC-24. Dieses Gen wurde von Masuzawa et al., J. Biochem. 112, 609-615 (1992), vollständig aufgeklärt.

Die nun identifizierte Isoform wurde MGC-24v genannt und als CD164 klassifiziert (Zannettino et al., "CD164 Workshop Panel Report", in: Leucocyte Typing VI, Kishimoto, T. et al., Garland Publishing, New York (im Druck)). Eine mit der CD164-spezifischen cDNA stabil transfizierte Zellinie wurde in das Screeningverfahren eingesetzt, das bei der Herstellung der Hybridomzellen, die den Antikörper 67D2 produzieren, verwendet wurde.

Beispiel 3: Kreuzblockierung CD164-spezifischer monoklonaler Antikörper

Drei verschiedene monoklonale, CD164-spezifische Antikörper wurden in ein Kreuzblockierungsexperiment eingesetzt, um zu analysieren, ob diese Antikörper ähnliche oder verschiedene Epitope auf dem CD164 Glykoprotein erkennen.

Die Antikörper waren der Antikörper 103B2, der Gegenstand der DE 195 30 272 C1 ist, der Antikörper 105A5, der Gegenstand der DE 195 30 273 C1 ist sowie der erfindungsgemäße Antikörper 67D2.

Als Antigen wurde die MOLM-1-Zelle eingesetzt (Matsuo Y., et al., "Establishment and Characterization of Novel Megakaryoblastoid Cell Line, MOLM-1, from a Patient with Chronic Myelogenous Leukemia (1991)", Human Cell, 4: 261-264). Diese Zellinie zeichnet sich durch eine hohe Expression von CD164 Glykoprotein auf seiner Zelloberfläche aus.

Die Antikörper 103B2, 105A5 und 67D2 sowie negative Kontrollantikörper mit jeweils gleichem Isotyp wurden getrennt voneinander für dreißig Minuten auf Eis mit MOLM-1-Zellen inkubiert. Dann wurden die Zellen gewaschen und entweder zur Positivkontrolle mit dem gleichen monoklonalen Antikörper oder mit einem der weiteren CD164 Antikörper inkubiert, um die Blockierung oder Nichtblockierung festzustellen. Danach wurden die Zellen mit einem PE-konjugierten Anti-IG-Antikörper inkubiert, der an die CD164-spezifischen monoklonalen Antikörper bindet. Danach wurden die Ansätze auf einem FACSCalibur Durchflußzytometer gemessen.

Tabelle 1 zeigt das Ergebnis dieses Tests, wobei die Blockierung in % % ausgedrückt wird. Der Prozentsatz an Blockierung wurde nach folgender Formel berechnet:

$$100 - [(\text{mittlere Fluoreszenz der mit dem getesteten Antikörper}$$

gefärbten Zellen nach Inkubation mit blockierendem Antikörper)

- (mittlere Fluoreszenz der mit einem negativen Kontrollantikörper

des jeweils passenden Isotyps gefärbten Zellen)] /

[(mittlere Fluoreszenz der mit dem Testantikörper gefärbten

Zellen nach Inkubation mit dem negativen Kontrollantikörper) -

(mittlere Fluoreszenz der mit einem negativen Kontrollantikörper

des jeweils passenden Isotyps gefärbten Zellen)] · 100.

TABELLE 1:

| KREUZBLOCKIERUNG CD164-SPEZIFISCHER MONOKLONALER ANTIKÖRPER | | | |
|---|---|---|---|
| CD164-spezifischer Testantikörper | % Blockierung der Bindung des CD 164-spezifischen Testantikörpers durch: | | |
| | 103B2 | 105A5 | 67D2 |
| 103B2 | - | 0% | 3,5% |
| 105A5 | 60,4 % | - | 0 % |
| 67D2 | 13,5 % | 3,5 % | |

Aus Tabelle 1 ist ersichtlich, daß nach Inkubation von MOLM-1-Zellen mit dem Antikörper 103B2 weder die Bindung des Antikörpers 105A5 noch die Bindung des Antikörpers 67D2 in signifikantem Ausmaß blockiert wird. Dies bedeutet, daß der Antikörper 103B2 ein Epitop erkennt, das sowohl von dem durch 105A5 als auch durch 67D2 erkannten Epitop verschieden ist.

Wären die Epitope nicht verschieden, so könnten 105A5 und 67D2 nicht mehr an die MOLM-1 Zellen binden.

Nach Inkubation der MOLM-1-Zellen mit dem Antikörper 105A5 war zwar eine partielle Blockierung der Bindung von 103B2 von ungefähr 60 % nachweisbar, für den Antikörper 67D2 ließ sich hingegen keine Blockierung nachweisen.

Wurden die MOLM-1-Zellen zuerst mit dem Antikörper 67D2 und danach jeweils mit dem Antikörper 103B2 oder 105A5 inkubiert, ließ sich in keinem der Fälle eine signifikante Blockierung der Bindung der zweiten Antikörper feststellen.

Der Antikörper 103B2 blockiert also die Bindung der Antikörper 105A5 und 67D2 an das CD164 Glykoprotein nicht.

Der Antikörper 105A5 blockiert die Bindung des Antikörpers 103B2 teilweise, die Bindung des Antikörpers 67D2 hingegen nicht.

Der Antikörper 67D2 blockiert die Bindung der Antikörper 103B2 und 105A2 an CD164 nicht.

Insgesamt ergibt sich, daß die drei verschiedenen CD164-spezifischen monoklonalen Antikörper unterschiedliche Epitope des CD164 Glykoproteins erkennen.

**Patentansprüche**

1. Monoklonaler Antikörper, der spezifisch gegen CD164 gerichtet ist, **dadurch gekennzeichnet, daß** er von Hybridomzellen produziert und freigesetzt wird, die unter der Nummer DSM ACC2303 bei der Deutschen Sammlung von Mikroorganismen und Zellkulturen GmbH, DSMZ, hinterlegt sind und die Bezeichnung "67D2" tragen.

2. Hybridomzellen, **dadurch gekennzeichnet, daß** sie unter der Nummer DSM ACC2303 bei der Deutschen Sammlung von Mikroorganismen und Zellkulturen GmbH, DSMZ, hinterlegt sind.

3. Verwendung eines Antikörpers nach Anspruch 1 zur diagnostischen *in vitro*-Behandlung von Tumoren, insbesondere von Magen-, Colon- und Brustkarzinomen.

4. Verwendung eines Antikörpers nach Anspruch 1 zur Herstellung eines Arzneimittels zur Behandlung von Tumoren, insbesondere von Magen-, Colon- und Brustkarzinomen.

5. Pharmazeutisches Mittel zur diagnostischen Behandlung von Tumoren, **dadurch gekennzeichnet, daß** es einen Antikörper gemäß Anspruch 1 enthält.

6. Pharmazeutisches Mittel nach Anspruch 5, **dadurch gekennzeichnet, daß** der Antikörper an ein zellulär zielgerichtetes Therapeutikum gekoppelt ist.

7. Pharmazeutisches Mittel zur therapeutischen Behandlung von Tumoren, **dadurch gekennzeichnet, daß** es einen Antikörper nach Anspruch 1 enthält.

8. Pharmazeutisches Mittel nach Anspruch 7, **dadurch gekennzeichnet, daß** der Antikörper an ein zellulär zielgerichtetes Diagnostikum gekoppelt ist.

9. Kit zum Nachweis von CD164, **dadurch gekennzeichnet, daß** er einen Antikörper nach Anspruch 1 enthält.

10. *In-vitro*-Verwendung eines Antikörpers nach Anspruch 1 zum Nachweis von hämatopoetischen Zellen.

11. Kit zum Nachweis von hämatopoetischen Zellen, **dadurch gekennzeichnet, daß** er einen Antikörper nach Anspruch 1 enthält.


**Claims**

1. A monoclonal antibody specific against CD164, **characterized in that** said antibody is produced and released by hybridoma cells that are deposited under No. DSM ACC2303 at the Deutsche Sammlung von Mikroorganismen und Zellkulturen GmbH, DSMZ, and said antibody is designated "67D2".

2. Hybridoma cells, **characterized in that** said cells are deposited under No. DSM ACC2303 at the Deutsche Sammlung von Mikroorganismen und Zellkulturen GmbH, DSMZ.

3. Use of an antibody according to claim 1 for diagnostic treatment *in vitro* of tumors, in particular of gastric, colon and breast carcinoma.

4. Use of an antibody according to claim 1 for the manufacture of a medicament for treatment of tumors, in particular of gastric, colon and breast carcinoma.

5. A pharmaceutical means for diagnostic treatment of tumors, **characterized in that** said means contains an antibody according to claim 1.

6. The pharmaceutical means according to claim 5, **characterized in that** said antibody is coupled to a cellularly targeted therapeutical agent.

7. A pharmaceutical means for therapeutic treatment of tumors, **characterized in that** said means contains an antibody according to claim 1.

8. The pharmaceutical means according to claim 7, **characterized in that** said antibody is coupled to a cellularly goal-oriented diagnostic agent.

9. Kit for the detection of CD164, **characterized in that** said kit contains an antibody according to claim 1.

10. Use of an antibody according to claim 1 in vitro for detection of haematopoietic cells.

11. Kit for the detection of haematopoietic cells, **characterized in that** said kit contains an antibody according to claim 1.

**Revendications**

1. Anticorps monoclonal dirigé spécifiquement contre CD164, **caractérisé en ce qu'**il est produit et libéré par des cellules d'hybridome, qui sont déposées sous le numméro DSM ACC2303 auprés de la Deutsche Sammlung von Mikroorganismen und Zellkutturen GmbH, DSMZ et qui portent la dénomination « 67D2».

2. Cellules d'hybridome, **caractérisées en ce qu'**elles sont déposées sous le numéro DSM ACC2303 auprès de la Deutsche Sammlung von Mikroorganismen und Zellkulturen GmbH, DSMZ.

3. Utilisation d'un anticorps selon la revendication 1 pour le traitement diagnostique *in vitro* de tumeurs, en particulier de carcinomes de l'estomac, du côlon et du sein.

4. Utilisation d'un anticorps selon la revendication 1 pour la fabrication d'un médicament pour le traitement de tumeurs, en particulier de carcinomes de l'estomac, du côlon et du sein.

5. Produit pharmaceutique pour fe traitement diagnostique de tumeurs, **caractérisé en ce qu'**il contient un anticorps selon la revendication 1.

6. Produit pharmaceutique salon la revendication 5, **caractérisé en ce que** l'anticorps est couplé à un produit thérapeutique cellulaire dirigé contre une cible.

7. Produit pharmaceutique pour le traitement thérapeutique de tumeurs, **caractérisé en ce qu'**il contient un anticorps selon la revendication 1.

8. Produit pharmaceutique selon la revendication 7, **caractérisé en ce que** l'anticorps est couplé à un produit de diagnostic cellulaire dirigé contre une cible.

9. Kit pour la détection de CD164, **caractérisé en ce qu'**il contient un anticorps selon la revendication 1.

10. Utilisation *in vitro* d'un anticorps selon la revendication 1 pour la détection de cellules hématopoïétiques.

11. Kit pour la détection de cellules hématopoïétiques, **caractérisé en ce qu'**il contient un anticorps selon la revendication 1.